# FASCICULE DE BREVET EUROPEEN

(11) **EP 0 835 105 B1**
(45) Date de publication et mention de la délivrance du brevet: **06.03.2002**
(21) Numéro de dépôt: 96924037.3
(22) Date de dépôt: 28.06.1996
(51) Int. Cl.: A61K 31/34, A61K 9/08, A61K 47/02, A61K 47/10, A61K 47/12, A61K 47/26, A61K 47/44

(54) **COMPOSITION PHARMACEUTIQUE D'AMIODARONE POUR ADMINISTRATION PARENTERALE**
PARENTERAL ANZUWENDENDE ARZNEIZUBEREITUNG ENTHALTEND AMIODARON
PHARMACEUTICAL AMIODARONE COMPOSITION FOR PARENTERAL DELIVERY

(30) Priorité: 30.06.1995 FR 9507939
(43) Date de publication de la demande: 15.04.1998
(73) Titulaire: SANOFI-SYNTHELABO, 75635 Paris Cédex 13 (FR)
(72) Inventeur: GAUTIER, Jean-Claude, 34830 Clapiers (FR); BELLAMY, Régine, 34160 Restinclières (FR)
(74) Mandataire: Le Guen, Gérard
(86) Numéro de dépôt international: FR9601010
(87) Numéro de publication internationale: WO9702031

(56) Documents cités:
- WO-A-93/19753
- CHEMICAL ABSTRACTS, vol. 97, no. 4, 26 Juillet 1982 Columbus, Ohio, US; abstract no. 28511, XP002015504 & J.CARDIOVASC.PHARMACOL., vol. 4, no. 3, 1982, pages 375-380, W.B.GOUGH ET AL.: "HYPOTENSIVE ACTION OF COMMERCIAL INTRAVENOUS AMIODARONE AND POLYSORBATE 80 IN DOGS."
- CHEMICAL ABSTRACTS, vol. 121, no. 14, 3 Octobre 1994 Columbus, Ohio, US; abstract no. 163827, XP002015505 & J.PARENTER.SCI.TECHNOL., vol. 47, no. 4, 1993, pages 161-165, G.H.WARD ET AL.: "STUDIES IN PHLEBITIS.VI:DILUTION-INDUCED PRECIPITATION OF AMIODARONE HCl."

## Description

La présente invention se rapporte, d'une manière générale, à une nouvelle composition pharmaceutique contenant comme principe actif un dérivé de benzoyl-benzofuranne

En particulier, l'invention concerne une composition pharmaceutique pour administration parentérale contenant, comme principe actif, le 2-n-butyl-3-[3,5-diiodo-4-(2-diéthylaminoéthoxy)-benzoyl]-benzofuranne également dénommé amiodarone, ou un de ses sels pharmaceutiquement acceptables de préférence son chlorhydrate.

Ce composé. connu pour ses propriétés bénéfiques sur le système cardiovasculaire, est largement utilisé à l'heure actuelle, sous forme de son chlorhydrate, comme agent antiarythmique et comme principe actif dans le traitement de fond de l'insuffisance coronarienne.

Il est disponible notamment sous forme de solution pour administration parentérale c'est-à-dire sous forme de solution injectable concentrée, capable d'être diluée en une solution pour administration par perfusion.

Toutefois, en raison de sa solubilité particulière, le chlorhydrate d'amiodarone pose certains problèmes lors de la mise au point d'une formulation pharmaceutique injectable qui soit à la fois concentrée, stable et diluable.

En effet, on a remarqué que la solubilité de ce sel dans l'eau dépend fortement de la température : à 20 °C elle est de 0,3 à 0,5 mg/ml tandis qu'à chaud, vers 60 °C, elle augmente de manière brutale jusqu'à dépasser 100 mg/ml.

En outre, l'étude en diffusion de lumière d'une solution aqueuse à 50 mg/ml de ce pnncipe actif montre des taux de diffusion notables qui tendent à prouver que, même à haute concentration, des structures colloïdales existent, les mesures de diamètre par corrélation de photons faisant d'ailleurs apparaître une population centrée sur 100 nm.

De même, en microscopie de contraste de phases. l'observation de solutions aqueuses concentrées de chlorhydrate d'amiodarone permet de distinguer une organisation supramoléculaire de type smectique.

A la suite de ces observations, on a émis l'hypothèse que ce principe actif solubilisé présente une structure micellaire ou plutôt bicontinue.

Cette "pseudo-solution" aqueuse obtenue par chauffage est assez stable. Toutefois, si on la dilue dans l'eau, elle devient brutalement turbide en dessous de 2mg/ml.

L'étude de telles solutions diluées, en diffusion de lumière, montre en effet l'apparition brutale de structures de gros diamètre (environ 10 000 nm) de type vésiculaire. responsables de la turbidité en dessous de cette concentration.

Lors d'une dilution, il apparaît par conséquent souhaitable de maintenir cette structure apparemment supramoléculaire de type bicontinu qui correspond à un état d'équilibre.

Le chlorhydrate d'amiodarone, sous forme de solution concentrée (50 mg/ml) est actuellement commercialisé en ampoule de 3 ml notamment pour administration injectable directe. Pour une utilisation en perfusion, le contenu de cette ampoule, qui répond à la formulation suivante (% en poids/volume)

| | |
|---|---|
| Chlorhydrate d'amiodarone | 5 % |
| Polysorbate 80 | 10% |
| Alcool benzylique | 2 % |
| Eau pour préparation injectable | 83% |

doit être ajouté à un volume réduit de sérum glucosé, en l'occurrence 250 ml, de manière à atteindre rapidement et à maintenir un taux sanguin thérapeutique.

Par dilution de solutions injectables à 50 mg/ml de chlorhydrate d'amiodarone comme indiqué ci-dessus, on constitue généralement des solutions parentérales de concentrations inférieures à 2 mg/ml en particulier des solutions de 0,5 à 2 mg/ml

Ainsi, l'administration de chlorhydrate d'amiodarone selon cette formulation injectable, fait appel à une solution aqueuse de ce principe actif contenant 100 fois sa limite de solubilité dans l'eau à température ambiante. D'autre part, pour une administration par perfusion la formulation en question doit être diluée de telle sorte que la concentration en principe actif après dilution se situe à l'intérieur même de la zone d'instabilité maximale comprise entre 0,5 mg/ml et 2 mg/ml environ, justifiant, par conséquent, la présence d'une teneur relativement élevée de polysorbate 80 comme agent de stabilisation

Toutefois, il est bien connu que le polysorbate 80, surtout à des concentrations non négligeables, peut, dans une certaine mesure, provoquer des effets physiologiques indésirables.

Il est donc important de pouvoir disposer d'une solution aqueuse d'amiodarone, par exemple sous forme de son chlorhydrate, à la fois concentrée et dépourvue des désavantages ci-dessus ou dont on en a fortement réduit l'incidence, tout en étant diluable à des concentrations utiles en thérapeutique.

On a déjà tenté de remédier à ces désavantages en n'apportant toutefois que des solutions partielles aux problèmes posés.

Par exemple, on a proposé, dans la demande de brevet international WO 93/19753, des compositions concentrées en amiodarone pour administration parentérale, c'est-à-dire des solutions injectables, préparées à chaud puis refroidies, contenant de 15 à 50 mg/ml de principe actif, plus particulièrement des solutions de 25 à 50 mg/ml, dans un système tampon acétate aqueux maintenant le pH de 3.2 à 4,6, plus généralement de 3,2 à 3.8

On y a mentionné en outre que dans un tampon phtalate (0,1 M; pH = 3,5 à 3,8), un précipité se produit lorsque la solution d'amiodarone chauffée est refroidie tandis qu'un gel se forme à tous les pH par refroidissement d'une solution de ce principe actif préalablement chauffée dans un tampon phosphate (0,1M; pH = 3,5 à 3,8).

On a également exemplifié, dans cette demande de brevet, des solutions diluées pour administration par perfusion contenant de 0,10 à 0,15 mg/ml d'amiodarone. Toutefois, la préparation de ces solutions diluées a été réalisée non pas à partir de solutions concentrées, telles que les solutions de 25 à 50 mg/ml exemplifiées, mais au départ d'une solution mère contenant de 10 à 15 mg/ml de principe actif dans un tampon acétate de pH = 3,8, introduite dans une solution saline / dextrose.

En pratique, pour un traitement par voie parentérale, deux solutions mères différentes sont par conséquent nécessaires, l'une concentrée pour administration par injection directe, l'autre peu concentrée pour dilution en une solution pour administration par perfusion veineuse.

Au surplus, les solutions diluées pour administration parentérale ainsi obtenues présentent des inconvénients majeurs qui les rendent difficilement utilisables voire inacceptables en thérapeutique En effet. compte tenu de la posologie journalière recommandée pour une administration de chlorhydrate d'amiodarone par perfusion veineuse à savoir de 5 à 20 mg/kg/jour et de leur taux élevé de dilution, les solutions exemplifiées dans la demande de brevet ci-dessus, contenant de 0,10 à 0,15 mg/ml de principe actif, devraient être administrées selon des volumes extrêmement importants, en moyenne de 4 à 5 l par jour, ce qui est totalement exclu en pratique médicale.

Des essais préliminaires effectués dans le cadre de la présente invention ont clairement montré les limites des solutions parentérales, concentrées ou diluées, proposées dans la demande de brevet citée ci-dessus

Par exemple, on a pu mettre en évidence que des solutions concentrées en chlorhydrate d'amiodarone, telles que des solutions de 15 à 50 mg/ml dans un tampon acétate de pH situé entre 3,2 et 3,8, ne peuvent être diluées dans l'eau saline glucosée au-delà de 1 mg/ml sans former des solutions très opalescentes voire laiteuses.

Par contre, si des solutions de 10 à 15 mg/ml de ce principe actif, peuvent être fortement diluées dans l'eau saline glucosée jusqu'à atteindre 0,10 à 0,15 mg/ml, il n'est toujours pas possible d'effectuer une dilution dans la zone de concentration particulièrement critique de 0,5 à 0,8 mg/ml sans apparition de fortes turbidités.

Enfin, ces solutions concentrées, préparées à chaud nécessitent le respect d'une température très précise, à savoir, 61°C ± 1°C, sous peine d'apparition d'une turbidité prononcée et la formation de compositions non reproductibles.

L'ensemble de ces résultats expliquent à la fois la dualité des solutions mères nécessaires pour une administration parentérale, selon la demande de brevet ci-dessus, ainsi que la dilution particulièrement élevée des compositions pour administration par perfusion proposées dans cette même demande de brevet.

La recherche d'une formulation injectable concentrée en amiodarone, de préférence sous forme d'un de ses sels pharmaceutiquement acceptables tel que son chlorhydrate, qui soit à la fois diluable à des concentrations thérapeutiquement acceptables et dépourvues des désavantages rapportés précédemment reste par conséquent d'un intérêt incontestable.

Or, on a maintenant découvert, de manière surprenante, selon l'invention, qu'il est possible de disposer de solutions aqueuses concentrées en amiodarone, en particulier sous forme de son chlorhydrate, de préférence des solutions de 30 à 50 mg/ml ou 3 à 5% en poids, à la fois utilisables en injection directe ou par perfusion après dilution de manière convenable, par exemple dans le sérum glucosé

Ainsi, on a pu mettre en évidence que l'ajout d'une quantité suffisante d'un agent tensio-actif à une solution d'amiodarone, de préférence sous forme d'un sel pharmaceutiquement acceptable tel que le chlorhydrate, dans une solution tampon appropriée permet de former des solutions concentrées stables et limpides en principe actif qui peuvent être diluées, si nécessaire, jusqu'à atteindre la zone critique de 0,5 à 2mg/ml ou 0,05 à 0,2% en poids de manière à former une composition pharmaceutique pour administration par perfusion

En conséquence, l'invention concerne une composition pharmaceutique pour administration parentérale comprenant
◆ de 1,5 à 8 % en poids de la composition finale, préférentiellement de 3 à 5 %, de principe actif constitué par l'amiodarone ou un de ses sels pharmaceutiquement acceptables, en particulier son chlorhydrate
◆ une solution tampon physiologiquement acceptable capable de maintenir le pH de la composition entre 2,4 et 3,8
◆ un agent tensio-actif hydrophile non lonique.

Dans la suite de la description de même que dans les revendications et sauf indications contraires, les pourcentages des différents constituants formant les compositions de l'invention à savoir le principe actif, le milieu tampon et l'agent tensio-actif, expriment tous des proportions pondérales à savoir des % en poids de la composition finale qu'elle soit concentrée ou diluée.
En général, on choisit la solution tampon parmi des solutions aqueuses physiologiquement acceptables capables à la fois de dissoudre le principe actif et de maintenir le pH des compositions entre 2,4 et 3,8 de préférence entre 3,2 et 3,8.
En fait, si des pH inféneurs à 2,4 ne peuvent être tolérés par le patient, des pH supérieurs à 3,8 se révèlent incompatibles puisque l'on y enregistre une instabilité des compositions concentrées en principe actif notamment en chlorhydrate d'amiodarone, même en présence d'un agent tensio-actif hydrophile non ionique, se traduisant par de la turbidité prononcée et même par la formation de gel.

Ainsi, des systèmes tampons utilisables dans le cadre de l'invention peuvent être, par exemple, une solution aqueuse d'acide acétique/acétate de métal alcalin, tel que l'acétate sodique ou potassique, capable de stabiliser le pH à environ 3,5, une solution aqueuse de tampon Mac Ilvaine, comprenant une association acide phosphonque/phosphate monopotassique/acide citrique, capable de stabiliser valablement le pH de 2,4 à 3,3 ou encore une solution aqueuse d'acide orthophosphorique/phosphate monométallique alcalin, par exemple le phosphate monosodique ou monopotassique ou une solution aqueuse de glycine/acide fort tel que l'acide chlorhydrique, ces solutions tampons pouvant efficacement maintenir le pH à environ 3,3.

Dans la majorité des cas, la force ionique de la solution tampon sera comprise entre 0,08 molaire et 0,3 molaire, de préférence entre 0,1 et 0,3 molaire, selon le système tampon choisi. Au-delà de 0,3 molaire, la concentration en sels dans le milieu risque de perturber la stabilité des compositions de l'invention et d'entraîner une hypertonie des formulations alors qu'à une concentration inférieure à 0,08 molaire l'effet tampon devient inexistant.

A titre d'exemple, on peut citer des solutions tampons aqueuses 0,1 à 0,15 molaire en acide orthophosphorique/phosphate monométallique alcalin par exemple phosphate monosodique ou monopotassique; 0,1 à 0,3 molaire en acide acétique/acétate de métal alcalin par exemple acétate sodique ou potassique ou 0,2 molaire en glycine/acide fort tel qu'acide chlorhydrique.

Par contre, des tampons tels qu'acide citrique/citrate sodique ou hydrogénophtalate potassique, pourtant capables de maintenir le pH dans la zone recherchée, se sont révélés inadaptés même en présence d'un tensio-actif selon l'invention, le principe actif n'étant pas soluble dans ces solutions tampons.

Quant à l'agent tensio-actif, on le choisit généralement parmi des composés hydrophiles non ioniques dont l'équilibre hydrophile/lipophile, communément défini par sa valeur "HLB" traduisant la proportion des groupements hydrophiles et des groupements lipophiles de la molécule selon le système de GRIFFIN W.C. [J.Soc. Cosm. Chem, 1, 311 (1949)], est compris entre 13 et 29, de préférence entre 13 et 17.

En général, cet agent tensio-actif hydrophile non ionique est incorporé dans les compositions parentérales de l'invention à raison de 0,5 % à 2 % de préférence à une concentration de 0,9 à 1,5 %.

Des agents tensio-actifs de ce type peuvent être par exemple un copolymère oxyde d'éthylène/oxyde de propylène tel que ceux commercialisés sous les marques PLURONIC® P 94 (HLB : 13,5) et PLURONIC® F 68 (HLB : 29), une huile de ricin polyéthoxylée telle que celle commercialisée sous la marque CREMOPHOR® EL (HLB : 13), un polysorbate éthoxylé tel que le polysorbate 80 (HLB : 15) commercialisé sous la marque TWEEN® 80 ou encore un hydroxystéarate de polyéthylène tel que l'hydroxystéarate de polyéthylène -660 (HLB : 13) commercialisé sous la marque SOLUTOL® HS 15.

Si nécessaire, les compositions de l'invention peuvent comprendre un ou plusieurs ingrédients supplémentaires notamment un agent conservateur ou protecteur tel qu'un agent bactéricide.

A titre d'exemple, on peut citer l'alcool benzylique en faible proportion c'est-à-dire jusqu'à 1 % en poids de la composition finale.

Les compositions de l'invention ainsi formées, se caractérisent par une stabilité importante qui permet à la fois de les conserver de manière durable et de les utiliser efficacement pour une administration par injection directe

A ce titre, des compositions préférées de l'invention pour administration par injection contiennent de 3 à 5% de principe actif tel que le chlorhydrate d'amiodarone, une solution tampon 0,1 molaire acide orthophosphorique/monophosphate de sodium et de 1,3 à 1,6% de polysorbate 80.

Les compositions pharmaceutiques de l'invention peuvent être obtenues, de manière classique, en chauffant à une température de 60°C à 70°C, un milieu formé d'une solution tampon appropriée, de l'agent tensio-actif hydrophile non ionique sélectionné et du principe actif puis en refroidissant jusqu'à la température ambiante ta composition pharmaceutique ainsi formée.

Cette composition d'aspect limpide peut être préparée à une température quelconque de la gamme considérée contrairement à la demande de brevet citée précédemment où la marge très étroite de 61°C ± 1°C doit être scrupuleusement respectée.

On a observé, en outre, dans le cadre de la présente invention que, pour une administration par perfusion veineuse, les compositions injectables de l'invention peuvent être diluées de manière à réduire la concentration en principe actif jusqu'à au moins un minimum de 0,05 % en poids de la composition diluée sans craindre l'apparition d'opalescence.

En conséquence. selon un autre de ses aspects, l'invention concerne une composition pharmaceutique pour administration parentérale comprenant :
◆ de 1,5 % à 8 %, en particulier de 3 à 5 %, d'un principe actif constitué d'amiodarone, de préférence sous forme d'un de ses sels pharmaceutiquement acceptables, en particulier son chlorhydrate
◆ une solution tampon et un agent tensio-actif hydrophile non ionique tels que les solutions tampons et agents tensio-actifs décrits précédemment pour les compositions concentrées injectables de l'invention,
et une quantité suffisante d'un diluant physiologiquement acceptable, la concentration totale en principe actif n'étant pas inférieure à 0,05 %.

De préférence, les compositions diluées de l'invention comprennent de 0,05% à 0,5 %, habituellement de 0,08 à 0,2 % de principe actif tandis que le diluant physiologiquement acceptable est formé, en général, de sérum physiologique contenant un hydrate de carbone tel que par exemple le glucose (ou dextrose) ou le sorbitol.

Les caractéristiques et avantages des compositions selon l'invention apparaîtront à la lumière de la description ci-après à partir de compositions données à titre d'exemples.

### I - Détermination de la turbidité de compositions selon l'invention

On a rapporté ci-dessous les résultats de différents essais effectués en vue de déterminer, par néphélométrie, la turbidité de solutions de chlorhydrate d'amiodarone selon l'invention, diluées ou non dans du sérum glucosé.

A cette fin, on a utilisé un turbidimètre de laboratoire capable de mesurer des turbidités comprises entre 0,001 et 1999 unités de turbidité néphélométrique (NTU).

Le test a consisté à diriger un faisceau lumineux ayant une longueur d'onde de 550 ± 50 nm de manière qu'il traverse de part en part la solution à étudier.

A l'aide de détecteurs, on a alors mesuré la lumière dispersée selon un angle de 90°, la lumière dispersée selon un angle de 30° par rapport à la direction de la lumière incidente et la lumière transmise au travers de la solution.

Les résultats ont été exprimés en NTU et interprétés comme suit :

| **Turbidité (T) (en NTU)** | **La solution est considérée comme :** |
|---|---|
| T < 3 | Limpide |
| 3 < T < 6 | Faiblement opalescente |
| 6 < T < 18 | Opalescente |
| 18 < T < 30 | Très opalescente |

### a) Formulation dans le milieu tampon acide acétique / acétate de sodium

Les résultats de turbidité d'une solution concentrée en chlorhydrate d'amiodarone de formulation suivante

| | |
|---|---|
| Chlorhydrate d'amiodarone | 5,00% |
| Milieu tampon acide acétique / acétate sodique (0,1 M , pH = 3,6) | 93,10% |
| PLURONIC ® F 68 | 1,90% |

ont été déterminés après 1 mois de stockage à différentes températures à savoir 5°C, 25°C et 35°C.
Les mêmes déterminations ont été effectuées dans les mêmes conditions au départ d'une solution identique diluée jusqu'à obtention d'une concentration de 0,1 % en chlorhydrate d'amiodarone dans du sérum glucosé à 5 % en poids.
Dans chaque cas, on a dosé la concentration en principe actif au moment de la détermination de la turbidité.
Les résultats obtenus sont reproduits ci-après :

| **Température de stockage (°C)** | **Concentration en chlorhydrate d'amiodarone (%)** | **Turbidité (NTU)** | **pH de la solution** |
|---|---|---|---|
| 5 | 4,998 | 3,4 | 3,52 |
| | 0,098 | 0,25 | 4,02 |
| 25 | 4,998 | 3,8 | 3,62 |
| | 0,102 | 0,27 | 4,06 |
| 35 | 4,998 | 4,1 | 3,63 |
| | 0,099 | 0,4 | 4,1 |

### b) Formulation dans le milieu tampon acide orthophosphorique / phosphate monosodique

Les mesures de turbidité de solutions de chlorhydrate d'amiodarone préparées à partir d'une composition concentrée de formulation suivante :

| | |
|---|---|
| Chlorhydrate d'amiodarone | 5,00% |
| Milieu tampon acide orthophosphorique/phosphate monosodique (0,1 M ; pH = 3,3) | 93,58% |
| Polysorbate 80 | 1,42% |

diluées jusqu'à obtention d'une concentration en chlorhydrate d'amiodarone de 0,10 % ; 0,06 % ou 0,05 % dans le sérum glucosé à 5 % en poids ont foumi les résultats suivants :

| **Concentration en chlorhydrate d'amiodarone (%)** | **Turbidité (NTU)** |
|---|---|
| 0.10 | 0,31 |
| 0,06 | 0,31 |
| 0,05 | 0,32 |

Aucune impureté n'a été détectée dans ces essais.
D'autres tests ont été réalisés à partir de solutions concentrées en chlorhydrate d'amiodarone de formulations suivantes dans le milieu tampon acide orthophosphorique/ phosphate monosodique après dilution jusqu'à obtention d'une concentration en chlorhydrate d'amiodarone de 0,05% dans le sérum glucosé à 5 % en poids.

| | |
|---|---|
| Chlorhydrate d'amiodarone | 5,00% |
| Milieu tampon acide orthophophorique/phosphate monosodique (0,1 M ; pH = 3,3) | 93,58% |
| Tensio-actif associé : | |
| CREMOPHOR® EL | |
| ou | 1,42% |
| SOLUTOL® HS 15 | |

Dans les deux cas, on a enregistré une turbidité de 0,4 NTU.

### II - Etudes comparatives

### a) Compositions concentrées en chlorhydrate d'amiodarone pour injection directe

Des études comparatives, réalisées entre des compositions concentrées en chlorhydrate d'amiodarone selon l'état de la technique représenté par la demande de brevet citée ci-dessus et une composition de l'invention, ont fourni les résultats rapportés ci-après :

| **Concentration en chlorhydrate d'amiodarone (%)** | **Milieu tampon (0,1M)** | **Tensio-actif** | **Turbidité (NTU)** |
|---|---|---|---|
| 1,0 | Acide acétique/acétate sodique (pH = 3,8) | - | 240 |
| 1,5 | Acide acétique/acétate sodique (pH = 3,8) | - | 19 |
| 5,0 | Acide acétique/acétate sodique (pH = 3,5) | Polysorbate 80 (0,95%) | 3,4 |

Ces résultats montrent que la composition de l'invention, contenant 5% de principe actif, présente une très faible turbidité contrairement aux compositions antérieures Comme la turbidité traduit généralement la présence de gros amas capables de provoquer des problèmes lors de l'injection et d'entrainer un manque de stabilité des solutions à administrer, les compositions de l'invention sont effectivement plus avantageuses que les compositions de l'état de la technique.

### b) Compositions diluées en chlorhydrate d'amiodarone pour perfusion veineuse

Des tests comparatifs analogues aux essais précédents ont été réalisés à partir de compositions obtenues par dilution de solutions concentrées en chlorhydrate d'amiodarone respectivement conformes à l'état de la technique représenté par la demande de brevet citée ci-dessus et à l'invention à l'aide de sérum glucosé à 5% en poids.
Les résultats, déterminés immédiatement après la dilution, sont rapportés ci-dessous :

| **Concentration en chlorhydrate d'amiodarone avant dilution (%)** | **Milieu tampon (0,1M)** | **Tensio-actif (%)** | **Turbidité (NTU) après dilution à :** | |
|---|---|---|---|---|
| | | | **0,075%** | **0,050%** |
| 1,0 | Acide acétique/acétate sodique (pH = 3,8) | - | 690 | 450 |
| 1,5 | Acide acétique/acétate sodique (pH = 3,8) | - | 650 | 580 |
| 5,0 | Acide acétique/acétate sodique (pH = 3,8) | - | 670 | 375 |
| 5,0 | Acide acétique/acétate sodique (pH = 3,5) | Polysorbate 80 (0,95%) | 0,50 | 0,66 |

Ces résultats montrent que si la composition concentrée selon l'invention peut être valablement diluée dans le sérum glucosé dans la zone de 0,050% à 0,075% par contre les compositions concentrées de l'art antérieur, diluées à ces mêmes concentrations, donnent naissance à des solutions d'aspect très opalescent voire laiteux
Une étude comparative a également été menée mettant en oeuvre des solutions préparées, à partir de solutions concentrées à 5% en chlorhydrate d'amiodarone. solutions aqueuses ou tamponnées, par dilution dans un sérum glucosé à 5 % en poids
Les résultats suivants ont été fournis

| **Milieu tampon (0,1M)** | **Tensio-actif (%) (%)** | **Turbidité (NTU) après dilution à :** | |
|---|---|---|---|
| | | 0,075% | 0,050% |
| Acide acétique/acétate sodique (pH= 3,8) | - | 670 | 375 |
| - | Polysorbate 80 (0,95%) | 360 | 245 |
| - | Polysorbate 80 (1,9 %) | 190 | 139 |
| Acide acétique/acétate sodique (pH=3,5) | Polysorbate 80 (0,95%) | 0,50 | 0,66 |

Ainsi, on observe qu'une solution concentrée en chlorhydrate d'amiodarone dans le milieu tampon seul ne peut être diluée valablement dans la zone critique sans l'apparition de turbidités très importantes.
Toutefois, une faible quantité de tensio-actif non ionique ajoutée à ce milieu tampon solubilisant autorise la dilution de la solution concentrée de chlorhydrate d'amiodarone obtenue alors que cette même quantité de tensio-actif non ionique ajoutée à une solution aqueuse ne permet pas de diluer la solution concentrée ainsi formée.
Ces résultats montrent clairement un effet de synergie exercé entre le milieu tampon et le tensio-actif non ionique.
Grâce à cet effet synergique mis en évidence au niveau des composants milieu tamponné / tensio-actif non lonique, on peut formuler des compositions parentérales notamment dans un milieu acide orthosphorique / phosphate monométallique alcalin, contrairement à l'état de la technique, de manière à constituer des compositions concentrées ou diluées en principe actif.
Les Exemples, non limitatifs suivants, illustrent les compositions de l'invention.

### EXEMPLE 1

### Composition concentrée injectable de chlorhydrate d'amiodarone

On prépare une composition pharmaceutique injectable d'un poids total de 100g et répondant à la formulation suivante :

| | |
|---|---|
| Chlorhydrate d'amiodarone | 5g |
| Milieu tampon acide orthophosphorique/phosphate monosodique (0,1M ; pH=3,3) | 93,575g |
| Polysorbate 80 | 1,425g |

par application du procédé décrit ci-dessous

On constitue d'abord une solution tampon 0,1M de pH = 3,3 en dissolvant 1,56g de phosphate monosodique (NaH₂ PO₄) dans 100 ml d'eau et en ajustant ensuite le pH à 3,3 par addition d'acide orthophosphorique molaire.

A 1,5 g de polysorbate 80, on ajoute alors 98,5 g de la solution tampon précédente puis on ajoute 95 g du milieu ainsi constitué à 5 g de chlorhydrate d'amiodarone.

Sous agitation, on chauffe alors l'ensemble au bain-marie entre 60 et 70°C pendant 15 minutes puis on laisse refroidir jusqu'à la température ambiante.

### EXEMPLES 2 à 14

### Compositions concentrées injectables de chlorhydrate d'amiodarone

En suivant la même méthode qu'à l'Exemple 1 mais en faisant varier notamment la nature de l'agent tensio-actif hydrophile non ionique, on a préparé les compositions suivantes d'un poids total de 100g.

| | | |
|---|---|---|
| EX 2 | Chlorhydrate d'amiodarone | 5g |
| | Milieu tampon acide acétique/acétate sodique (0,1M ; pH = 3,6) | 93,1g |
| | PLURONIC® F68 | 1,9g |
| EX.3 | Chlorhydrate d'amiodarone | 5g |
| | Milieu tampon acide orthophosphoriquelphosphate monosodique (0,1 M ; pH = 3,3) | 93,575g |
| | CREMOPHOR® EL | 1,425g |
| EX 4 | Chlorhydrate d'amiodarone | 5g |
| | Milieu tampon acide orthophosphorique/phosphate monosodique (0,1M ; pH = 3,3) | 93,575g |
| | SOLUTOL® HS 15 | 1,425g |
| EX.5 | Chlorhydrate d'amiodarone | 5g |
| | Milieu tampon acide orthophosphorique/phosphate monosodique (0,1M ; pH = 3,3) | 93,575g |
| | PLURONIC® P94 | 1,425g |
| EX.6 | Chlorhydrate d'amiodarone | 5g |
| | Milieu tampon acide orthophosphorique/phosphate monosodique (0,15M ; pH = 3,3) | 93,575g |
| | Polysorbate 80 | 1,425g |
| EX.7 | Chlorhydrate d'amiodarone | 5g |
| | Milieu tampon acide acétique/acétate sodique (0,3M ; pH = 3,5) | 93,575g |
| | Polysorbate 80 | 1,425g |
| EX.8 | Chlorhydrate d'amiodarone | 5g |
| | Milieu tampon glycine/acide chlorhydrique (0,2M ; pH = 3,3) | 94,059 |
| | Polysorbate 80 | 0,95g |
| EX.9 | Chlorhydrate d'amiodarone | 5g |
| | Milieu tampon acide acétique/acétate sodique (0,1 M ; pH = 3,5) | 94,05g |
| | SOLUTOL® HS 15 | 0,95g |
| EX.10 | Chlorhydrate d'amiodarone | 5g |
| | Milieu tampon acide acétique/acétate sodique (0,1 M ; pH = 3,5) | 94,05g |
| | CREMOPHOR® EL | 0,95g |
| EX.11 | Chlorhydrate d'amiodarone | 5g |
| | Milieu tampon acide acétique/acétate sodique (0,1M ; pH = 3,5) | 93,575g |
| | PLURONIC® P 94 | 1,425g |
| EX.12 | Chlorhydrate d'amiodarone | 5g |
| | Milieu tampon Mac Ilvaine (pH = 3,3) | 94,059 |
| | Polysorbate 80 | 0,95g |
| EX 13 | Chlorhydrate d'amiodarone | 5g |
| | Milieu tampon Mac Ilvaine (pH = 2,4) | 94.05g |
| | Polysorbate 80 | 0,95g |
| EX.14 | Chlorhydrate d'amiodarone | 1,5g |
| | Milieu tampon acide orthophosphorique/phosphate monosodique (0,1 M ; pH = 3,3) | 97,0225g |
| | Polysorbate 80 | 1,4775g |
| EX.15 | Chlorhydrate d'amiodarone | 3g |
| | Milieu tampon acide orthophosphonque/phosphate monosodique (0,1 M ; pH = 3,3) | 95,545g |
| | Polysorbate 80 | 1,455g |
| EX.16 | Chlorhydrate d'amiodarone | 8g |
| | Milieu tampon acide acétique/acétate sodique (0,1 M ; pH = 3,5) | 90,62g |
| | Polysorbate 80 | 1,38g |

### EXEMPLE 17

### Unité de dosage pour administration injectable de chlorhydrate d'amiodarone

On introduit dans une ampoule en verre stérilisée, 3 ml d'une composition stérilisée de chlorhydrate d'amiodarone telle que préparée à l'Exemple 1. On scelle alors l'ampoule sous aseptie de manière à constituer une unité de dosage contenant 50 mg/ml de chlorhydrate d'amiodarone ou 5% de ce principe actif.

### EXEMPLE 18

### Composition diluée de chlorhydrate d'amiodarone pour perfusion

On prépare une solution parentérale pour perfusion veineuse en ajoutant à 250 ml de solution injectable isotonique de glucose à 5 % en poids, 3 ml d'une composition injectable de chlorhydrate d'amiodarone à 5% selon l'Exemple 1 ou le contenu d'une ampoule selon l'Exemple 17.

De cette manière, on constitue une solution parentérale parfaitement limpide contenant 0,6 mg/ml de chlorhydrate d'amiodarone ou 0,06% de ce principe actif.

### EXEMPLE 19

### Composition diluée de chlorhydrate d'amiodarone pour perfusion

On prépare une solution parentérale pour perfusion veineuse, en introduisant dans 250 ml de solution injectable isotonique de glucose à 5 % en poids, 6 ml d'une composition injectable de chlorhydrate d'amiodarone à 5% selon l'Exemple 1 ou le contenu de deux ampoules selon l'Exemple 17.

De cette manière, on obtient une solution parentérale parfaitement limpide contenant 1,2 mg/ml de chlorhydrate d'amiodarone ou 0,12% de ce principe actif.

### EXEMPLE 20

### Composition diluée de chlorhydrate d'amiodarone pour perfusion

On prépare des solutions parentérales pour perfusion veineuse contenant de 0,5 à 5 mg/ml de chlorhydrate d'amiodarone, soit 0,05% à 0,5% de ce principe actif, par introduction d' un volume approprié d'une composition concentrée injectable selon les Exemples 2 à 16 dans une solution injectable isotonique de glucose à 5 % en poids.

Les solutions parentérales obtenues sont parfaitement limpides.

## Revendications

1. Composition pharmaceutique pour administration parentérale, **caractérisée en ce qu'**elle comprend :
◆ de 1,5 à 8 % en poids d'un principe actif constitué par l'amiodarone ou un de ses sels pharmaceutiquement acceptables
◆ une solution tampon physiologiquement acceptable capable de solubiliser le principe actif et de maintenir le pH de la composition entre 2,4 et 3,8
◆ un agent tensio-actif hydrophile non ionique.

2. Composition pharmaceutique selon la Revendication 1 **caractérisée en ce qu'**elle contient de 3 à 5% de principe actif.

3. Composition pharmaceutique selon la Revendication 1 ou 2 **caractérisée en ce que** la solution tampon est une solution aqueuse d'acide acétique/acétate de métal alcalin

4. Composition pharmaceutique selon la Revendication 1 ou 2 **caractérisée en ce que** la solution tampon est une solution aqueuse d'acide orthophosphorique/phosphate monométallique alcalin.

5. Composition pharmaceutique selon la Revendication 1 ou 2 **caractérisée en ce que** la solution tampon est une solution aqueuse de tampon Mac Ilvaine.

6. Composition pharmaceutique selon la Revendication 1 ou 2 **caractérisée en ce que** la solution tampon est une solution aqueuse de glycine/acide fort.

7. Composition pharmaceutique selon la Revendication 3 **caractérisée en ce que** la solution tampon d'acide acétique/acétate de métal alcalin maintient le pH à 3,5.

8. Composition pharmaceutique selon la Revendication 4 **caractérisée en ce que** la solution tampon d'acide orthophosphorique/phosphate monométallique alcalin maintient le pH à 3,3.

9. Composition pharmaceutique selon la Revendication 5 **caractérisée en ce que** la solution de tampon Mac Ilvaine maintient le pH de 2,4 à 3.3.

10. Composition pharmaceutique selon la Revendication 6 **caractérisée en ce que** la solution tampon de glycine/acide fort maintient le pH à 3,3.

11. Composition pharmaceutique selon une des Revendications 1 à 10 **caractérisée en ce que** la force ionique de la solution tampon est comprise entre 0,08 molaire et 0,3 molaire.

12. Composition pharmaceutique selon une des Revendications 1 à 3, 7 ou 11 **caractérisée en ce que** la solution tampon est une solution aqueuse 0,1 à 0,3 molaire en acide acétique/acétate de métal alcalin.

13. Composition pharmaceutique selon une des Revendications 1, 2, 4, 8 ou 11 **caractérisée en ce que** la solution tampon est une solution aqueuse 0,1 à 0,15 molaire en acide orthophosphorique/monophosphate de métal alcalin.

14. Composition pharmaceutique selon une des Revendications 1, 2, 6, 10 ou 11 **caractérisée en ce que** la solution tampon est une solution aqueuse 0,2 molaire en glycine/acide fort.

15. Composition pharmaceutique selon la revendications 3, 4, 7 ou 8, **caractérisée en ce que** le métal alcalin est le sodium ou le potassium.

16. Composition pharmaceutique selon la revendication 6 ou 10, **caractérisée en ce que** l'acide fort est l'acide chlorhydrique.

17. Composition pharmaceutique selon une des Revendications 1 à 16, **caractérisée en ce que** l'agent tensio-actif est choisi parmi des composés hydrophiles non ioniques dont la valeur HLB est comprise entre 13 et 29

18. Composition pharmaceutique selon la Revendication 17, **caractérisée en ce que** l'agent tensio-actif est choisi parmi des composés hydrophiles non ioniques dont la valeur HLB est comprise entre 13 et 17.

19. Composition pharmaceutique selon la Revendication 17 ou 18 **caractérisée en ce que** l'agent tensio-actif est utilisé à raison de 0,5 à 2 % en poids.

20. Composition pharmaceutique selon une des Revendications 17 à 19 **caractérisée en ce que** l'agent tensio-actif est un copolymère oxyde d'éthylène/oxyde de propylène, une huile de ricin polyéthoxylée, un polysorbate éthoxylé ou un hydroxystéarate de polyéthylène.

21. Composition pharmaceutique selon la Revendication 20 **caractérisée en ce que** l'agent tensio-actif est choisi parmi les produits commerciaux portant les marques suivantes :
PLURONIC® F68, PLURONIC® P 94, CREMOPHOR® EL. TWEEN® 80 ou SOLUTOL® HS 15.

22. Composition pharmaceutique selon une des Revendications 1 à 21 caracténsée en ce qu'elle contient en outre un agent conservateur ou protecteur.

23. Composition pharmaceutique selon la Revendication 22 **caractérisée en ce que** l'agent conservateur ou protecteur est l'alcool benzylique.

24. Composition pharmaceutique pour administration parentérale, **caractérisée en ce qu'**elle comprend une composition selon l'une quelconque des revendications 1 à 23 et une quantité suffisante d'un diluant physiologiquement acceptable, la concentration finale en principe actif n'étant pas inférieure à 0,05% en poids de la composition diluée

25. Composition pharmaceutique selon la Revendication 24 **caractérisée en ce que** la concentration finale en principe actif est comprise entre 0,05 % et 0,5% en poids de la composition diluée.

26. Composition pharmaceutique selon la Revendication 24 ou 25 **caractérisée en ce que** le diluant physiologiquement acceptable est formé de sérum physiologique contenant un hydrate de carbone

27. Composition pharmaceutique selon la Revendication 26 **caractérisée en ce que** l'hydrate de carbone est le glucose.

28. Composition pharmaceutique selon une des Revendications 1 à 27 **caractérisée en ce que** le principe actif est le chlorhydrate d'amiodarone.

29. Composition pharmaceutique pour administration parentérale selon la revendication 1 **caractérisée en ce qu'**elle comprend de 3 à 5 % en poids de chlorhydrate d'amiodarone, une solution tampon 0,1 molaire d'acide orthophosphorique/monophosphate de sodium et de 1,3 à 1,6 % en poids de polysorbate 80.

30. Composition pharmaceutique pour administration parentérale, **caractérisée en ce qu'**elle comprend une composition selon la revendication 29 et une quantité efficace de sérum physiologique glucosé, la concentration finale en chlorhydrate d'amiodarone n'étant pas inférieure à 0,05% en poids de la composition diluée.

31. Composition pharmaceutique selon la Revendication 30 **caractérisée en ce qu'**elle contient de 0,05 à 0,5% en poids de chlorhydrate d'amiodarone.

## Patentansprüche

1. Pharmazeutische Zubereitung zur parenteralen Verabreichung, **dadurch gekennzeichnet, daß** sie:
◆ 1.5 bis 8 Gew.-% Amiadaron oder eines pharmazeutisch annehmbaren Salzes davon als Wirkstoff,
◆ eine physiologische Pufferlösung, welche den Wirkstoff zu lösen und den pH-Wert der Zubereitung zwischen 2.4 und 3.8 zu halten vermag, und
◆ ein nichtionisches, hydrophiles oberflächenaktives Mittel umfaßt.

2. Pharmazeutische Zubereitung nach Anspruch 1, **dadurch gekennzeichnet, daß** sie 3 bis 5 % des Wirkstoffs enthält.

3. Pharmazeutische Zubereitung nach Anspruch 1 oder 2, **dadurch gekennzeichnet, daß** die Pufferlösung eine wäßrige Essigsäure/Alkalimetallacetat-Lösung ist.

4. Pharmazeutische Zubereitung nach Anspruch 1 oder 2, **dadurch gekennzeichnet, daß** die Pufferiösung eine wäßrige Orthophosphorsäure/Monoalkalimetallphosphat-Lösung ist.

5. Pharmazeutische Zubereitung nach Anspruch 1 oder 2, **dadurch gekennzeichnet, daß** die Pufferlösung eine wäßrige Mac Ilvaine-Pufferlösung ist.

6. Pharmazeutische Zubereitung nach Anspruch 1 oder 2, **dadurch gekennzeichnet, daß** die Pufferlösung eine wäßrige Lösung von Glycin und einer starken Säure ist.

7. Pharmazeutische Zubereitung nach Anspruch 3, **dadurch gekennzeichnet, daß** die Essigsäure/Alkalimetallacetat-Pufferlösung den pH-Wert bei 3,5 hält.

8. Pharmazeutische Zubereitung nach Anspruch 4, **dadurch gekennzeichnet, daß** die Orthophosphorsäure/Monoalkalimetallphosphat-Pufferlösung den pH-Wert bei 3,3 hält.

9. Pharmazeutische Zubereitung nach Anspruch 5, **dadurch gekennzeichnet, daß** die Mac Ilvaine-Pufferlösung den pH-Wert bei 2,4 bis 3,3 hält.

10. Pharmazeutische Zubereitung nach Anspruch 6, **dadurch gekennzeichnet, daß** die Pufferlösung aus Glycin und der starken Säure den pH-Wert bei 3,3 hält.

11. Pharmazeutische Zubereitung nach einem der Ansprüche 1 bis 10, **dadurch gekennzeichnet, daß** die Ionenstärke der Pufferlösung zwischen 0,08 molar und 0,3 molar liegt.

12. Pharmazeutische Zubereitung nach einem der Ansprüche 1 bis 3. 7 oder 11. **dadurch gekennzeichnet, daß** die Pufferlösung eine 0,1 bis 0,3 molare Essigsäure/Alkalimetallacetat-Lösung ist.

13. Pharmazeutische Zubereitung nach einem der Ansprüche 1, 2, 4, 8 oder 11, **dadurch gekennzeichnet, daß** die Pufferlösung eine wäßrige 0,1 bis 0,15 molare Alkalimetallmonophosphat-Lösung ist.

14. Pharmazeutische Zubereitung nach einem der Ansprüche 1, 2, 6, 10 oder 11. **dadurch gekennzeichnet, daß** die Pufferlösung eine 0,2 molare Lösung an Glycin und der starken Säure ist.

15. Pharmazeutische Zubereitung nach den Ansprüchen 3, 4, 7 oder 8, **dadurch gekennzeichnet, daß** das Alkalimetall Natrium oder Kalium ist.

16. Pharmazeutische Zubereitung nach Anspruch 6 oder 10, **dadurch gekennzeichnet, daß** die starke Säure Chlorwasserstoffsäure ist.

17. Pharmazeutische Zubereitung nach einem der Ansprüche 1 bis 16, **dadurch gekennzeichnet, daß** das oberflächenaktive Mittel aus nichtionischen hydrophilen Verbindungen mit einem HLB-Wert zwischen 13 und 29 ausgewählt ist.

18. Pharmazeutische Zubereitung nach Anspruch 17, **dadurch gekennzeichnet, daß** das oberflächenaktive Mittel aus nichtionischen hydrophilen Verbindungen mit einem HLB-Wert zwischen 13 und 17 ausgewählt ist.

19. Pharmazeutische Zubereitung nach Anspruch 17 oder 18, **dadurch gekennzeichnet, daß** das oberflächenaktive Mittel in einer Menge von 0,5 bis 2 Gew.-% vorhanden ist.

20. Pharmazeutische Zubereitung nach einem der Ansprüche 17 bis 19, **dadurch gekennzeichnet, daß** das oberflächenaktive Mittel ein Ethylenoxid/Propylenoxid-Copolymer, ein polyethoxyliertes Rizinusöl, ein ethoxyliertes Polysorbat oder ein Polyethylenhydroxystearat ist.

21. Pharmazeutische Zubereitung nach Anspruch 20, **dadurch gekennzeichnet, daß** das oberflächenaktive Mittel aus den handelsüblichen Produkten ausgewählt ist, die die folgenden Marken tragen:
PLURONIC® F68, PLURONIC® P 94, CREMOPHOR® EL, TWEEN® 80 oder SOLUTOL® HS 15.

22. Pharmazeutische Zubereitung nach einem der Ansprüche 1 bis 21, **dadurch gekennzeichnet, daß** sie zusätzlich ein Konservierungsmittel oder Schutzmittel enthält.

23. Pharmazeutische Zubereitung nach Anspruch 22, **dadurch gekennzeichnet, daß** das Konservierungs- oder Schutzmittel Benzylalkohol ist.

24. Pharmazeutische Zubereitung zur parenteralen Verabreichung, **dadurch gekennzeichnet, daß** sie eine Zubereitung nach einem der Ansprüche 1 bis 23 und eine wirksame Menge eines physiologisch verträglichen Verdünnungsmittels umfaßt, wobei die Endkonzentration des Wirkstoffs nicht weniger als 0.05 Gew.-% der verdünnten Zubereitung ausmacht.

25. Pharmazeutische Zubereitung nach Anspruch 24, **dadurch gekennzeichnet, daß** die Endkonzentration des Wirkstoffs zwischen 0.05 % und 0,5 Gew.-% liegt.

26. Pharmazeutische Zubereitung nach Anspruch 24 oder 25, **dadurch gekennzeichnet, daß** das physiologisch verträgliche Verdünnungsmittel aus physiologischem Serum, das ein Kohlenhydrat enthält, gebildet ist.

27. Pharmazeutische Zubereitung nach Anspruch 26, **dadurch gekennzeichnet, daß** das Kohlenhydrat Glucose ist.

28. Pharmazcutische Zubereitung nach einem der Ansprüche 1 bis 27, **dadurch gekennzeichnet, daß** das Wirkstoff Amiodaron-Hydrochlorid ist.

29. Pharmazeutische Zubereitung für die parenterale Verabreichung nach Anspruch 1, **dadurch gekennzeichnet, daß** sie 3 bis 5 Gew.-% Amiodaron-Hydrochlorid, eine 0,1 molare Orthophosphorsäure/Natriummonophosphat-Pufferlösung und 1.3 bis 1,6 Gew.-% Polysorbat 80 enthält.

30. Pharmazeutische Zubereitung für die parenterale Verabreichung, **dadurch gekennzeichnet, daß** sie eine Zubereitung nach Anspruch 29 und eine wirksame Menge Glucose-haltigen physiologischen Serums umfaßt, wobei die Endkonzentration an Amiodaron-Hydrochlorid nicht weniger als 0,05 Gew.-% der verdünnten Zubereitung beträgt.

31. Pharmazeutische Zubereitung nach Anspruch 30. **dadurch gekennzeichnet, daß** sie 0,05 bis 0,5 Gew.-% Amiodaron-Hydrochlorid enthält.

## Claims

1. Pharmaceutical composition for parenteral administration, **characterised in that** it comprises:
• 1.5 to 8% by weight of an active ingredient consisting of amiodarone or one of the pharmaceutically acceptable salts thereof
• a physiologically acceptable buffer solution capable of rendering the active ingredient soluble and maintaining the pH of the composition between 2.4 and 3.8
• a non-ionic hydrophilic surfactant.

2. Pharmaceutical composition according to claim 1, **characterised in that** it contains from 3 to 5% of active ingredient.

3. Pharmaceutical composition according to claim 1 or 2, **characterised in that** the buffer solution is an aqueous solution of acetic acid/alkali metal acetate.

4. Pharmaceutical composition according to claim 1 or 2, **characterised in that** the buffer solution is an aqueous solution of orthorphosphoric acid/alkali monometal phosphate.

5. Pharmaceutical composition according to claim 1 or 2, **characterised in that** the buffer solution is an aqueous solution of MacIlvaine buffer.

6. Pharmaceutical composition according to claim 1 or 2, **characterised in that** the buffer solution is an aqueous solution of glycine/strong acid.

7. Pharmaceutical composition according to claim 3, **characterised in that** the buffer solution of acetic acid/alkali metal acetate maintains the pH at 3.5.

8. Pharmaceutical composition according to claim 4, **characterised in that** the buffer solution of orthophosphoric acid/alkali monometal phosphate maintains the pH at 3.3.

9. Pharmaceutical composition according to claim 5, **characterised in that** the MacIlvaine buffer solution maintains the pH at 2.4 to 3.3.

10. Pharmaceutical composition according to claim 6, **characterised in that** the buffer solution of glycine/strong acid maintains the pH at 3.3.

11. Pharmaceutical composition according to one of claims 1 to 10, **characterised in that** the ionic force of the buffer solution is between 0.08 molar and 0.3 molar.

12. Pharmaceutical composition according to one of claims 1 to 3, 7 or 11, **characterised in that** the buffer solution is a 0.1 to 0.3 molar aqueous solution of acetic acid/alkali metal acetate.

13. Pharmaceutical composition according to one of claims 1, 2, 4, 8 or 11, **characterised in that** the buffer solution is a 0.1 to 0.15 molar aqueous solution of orthophosphoric acid/alkali metal monophosphate.

14. Pharmaceutical composition according to one of claims 1, 2, 6, 10 or 11, **characterised in that** the buffer solution is a 0.2 molar aqueous solution of glycine/strong acid.

15. Pharmaceutical composition according to claims 3, 4, 7 or 8, **characterised in that** the alkali metal is sodium or potassium.

16. Pharmaceutical composition according to claim 6 or 10, **characterised in that** the strong acid is hydrochloric acid.

17. Pharmaceutical composition according to one of claims 1 to 16, **characterised in that** the surfactant is selected from among the non-ionic hydrophilic compounds having an HLB value of between 13 and 29.

18. Pharmaceutical composition according to claim 17, **characterised in that** the surfactant is selected from among the non-ionic hydrophilic compounds having an HLB value of between 13 and 17.

19. Pharmaceutical composition according to claim 17 or 18, **characterised in that** the surfactant is used in an amount of 0.5 to 2% by weight.

20. Pharmaceutical composition according to one of claims 17 to 19, **characterised in that** the surfactant is a copolymer of ethylene oxide/propylene oxide, a polyethoxylated castor oil, an ethoxylated polysorbate or a polyethylene hydroxystearate.

21. Pharmaceutical composition according to claim 20, **characterised in that** the surfactant is selected from among the commercial products bearing the following trade marks;
PLURONIC® F68, PLURONIC® P 94, CREMOPHOR® EL, TWEEN® 80 or SOLUTOL® HS 15.

22. Pharmaceutical composition according to one of claims 1 to 21, **characterised in that** it further contains a preservative or protective agent.

23. Pharmaceutical composition according to claim 22, **characterised in that** the preservative or protective agent is benzyl alcohol.

24. Pharmaceutical composition for parenteral administration, **characterised in that** it comprises a composition according to any one of claims 1 to 23 and a sufficient quantity of a physiologically acceptable diluent, the final concentration of active ingredient being not less than 0.05% by weight of the diluted composition.

25. Pharmaceutical composition according to claim 24, **characterised in that** the final concentration of active ingredient is between 0.05% and 0.5% by weight of the dilute composition.

26. Pharmaceutical composition according to claim 24 or 25, **characterised in that** the physiologically acceptable diluent is formed by physiological serum containing a carbohydrate.

27. Pharmaceutical composition according to claim 26, **characterised in that** the carbohydrate is glucose.

28. Pharmaceutical composition according to one of claims 1 to 27, **characterised in that** the active ingredient is amiodarone hydrochloride.

29. Pharmaceutical composition for parenteral administration according to claim 1, **characterised in that** it comprises 3 to 5% by weight of amiodarone hydrochloride, a 0.1 molar buffer solution of orthophosphoric acid/sodium monophosphate and 1.3 to 1.6% by weight of polysorbate 80.

30. Pharmaceutical composition for parenteral administration, **characterised in that** it comprises a composition according to claim 29 and an effective amount of glucose-containing physiological serum, the final concentration of amiodarone hydrochloride being not less than 0.05% by weight of the diluted composition.

31. Pharmaceutical composition according to claim 30, **characterised in that** it contains 0.05 to 0.5% by weight of amiodarone hydrochloride.
